# EUROPEAN PATENT APPLICATION

(11) **EP 1 588 705 A1**
(43) Date of publication of application: **26.10.2005**
(21) Application number: 04009431.0
(22) Date of filing: 21.04.2004
(51) Int. Cl.: A61K 31/37, A61K 31/7048, A61P 25/00, A61P 25/28

(54) **Use of coumarin derivatives**

(71) Applicant: INTE:LIGAND Software-Entwicklungs und Consulting GmbH, 2344 Maria Enzersdorf (AT); Leopold-Franzens-Universität Innsbruck, 6020 Innsbruck (AT)
(72) Inventor: Stuppner, Hermann, Prof. Dr., 6091 Götzens (AT); Langer, Thierry, Dr., 6157 Ranggen (AT); Prast, Helmut, Dr., 6020 Innsbruck (AT); Rollinger, Judith, Dr., 6020 Innsbruck (AT); Wolber, Gerhard, Dr., 6840 Götzis (AT)
(74) Representative: Grünecker, Kinkeldey, Stockmair & Schwanhäusser Anwaltssozietät

(57) **Abstract**

The present invention relates to the use of a coumarin derivative having a coumarin skeleton of formula I 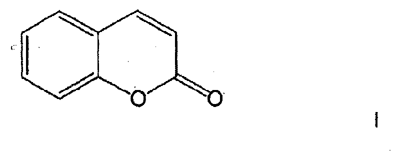 wherein the coumarin skeleton is substituted by one OR¹ and one OR²
whereby
R¹ is C₁ to C₆ alkyl, C₃ to C₇ cycloalkyl or C₂ to C₆ alkenyl and
R² is hydrogen or a sugar moiety, preferably scopoletin or scopolin for the preparation of a medicament for the prophylaxis of treatment of disorders which involve low levels of acetylcholine in the brain.
The coumarin derivatives of formula I inhibit acetylcholinesterase *in vitro* and enhance acetylcholine concentration in the brain *in vivo*, and therefore, can ameliorate disorders connected with deficits in learning and memory functions like Alzheimer's disease, senile dementia, ataxia, myasthenia gravis and Parkinson's disease.

## Description

The present invention is directed to the use of coumarin derivatives, in particular disubstituted coumarin derivatives, for the treatment of disorders involving low levels of acetylcholine.

The potency of natural products to treat and cure human diseases is well known and has been for living memory. Even today, in the century of combinatorial chemistry, secondary metabolites of plants, fungi, marine- and micro-organism are still an important source for the development of new drugs. Approximately one-third of the top-selling drugs in the world are derived from compounds of natural origin. Their potential has also been successfully demonstrated in the field of Alzheimer's disease (AD). Although the aetiology of AD is still unknown, its neuropathological occurrence associated with memory loss is consistent with the presence of numerous plaques in a cholinergic deficiency due to the degeneration or atrophy of cholinergic neurons in the basal forebrain. At present, the cholinesterase inhibition is the mainstay of treatment for AD and also serves as a promising strategy for the treatment of senile dementia, ataxia, myasthenia gravis and Parkinson's disease. Acetylcholinesterase (AChE) inhibitory drugs increase the effectiveness of cholinergic transmittions by inhibiting the metabolic hydrolysis of acetylcholine. In addition, evidence has been presented that AChE could play a key role in accelerating amyloid β-peptide (Aβ) plaques deposition. Since Aβ is the main component of the senile plaques found in the brain of AD patients, any compounds able to inhibit the plaques aggregation might be regarded as potentially useful in the treatment of AD.

Within the wide range of natural products Physostigmine, and indol-alkaloid from the seeds of *Physostigma venenosum* BALFOUR, turned out to be as one of the first principle anticholinesterases. However, it only shows a modest improvement in the cognitive function of Alzheimer's patients due to its poor brain penetration, oral bioavailability and pharmacokinetic parameters. Alkaloids of a number of Amaryllidaceae have been screened for AChE activity and one of the most potent of these emerged as galanthamine form *Galathus nivalis* L. which is now marked for the treatment of AD in Austria (Reminyl®). The alkaloid is selective, long acting, reversible, and produces beneficial cognitive effects inpatients. In spite of the definite potential of some natural products to enhance the cholinergic function in the CNS by their anti-cholinesterase effect there is still an unresolved problem of targeting selection from the multitude and chemical diversity of the natural products.

Coumarin derivatives have also been described in several publications as inhibitors of acetylcholinesterases. Choudhary, M.I., et al., describe in *Planta Medica* (2002), 68(1) 81-83, the isolation of coumarin derivatives, namely, murranganone and paniculatin and their inhibition of acetylcholinesterase and butyrylcholinesterase.

Simeon-Rudolf, V., et al., in *Chemico-Biological Interactions* 119-120 (1999) 119-128, describe the inhibition of recombinant mouse wild type acetylcholinesterase and butyrylcholinesterase by the coumarin derivative 3-chloro-7-hydroxy-4-methylcoumarin.

Coumarin derivatives as dual inhibitors of acetylcholinesterase and monoamine oxidase, whereby the coumarins are 3,4,7-substituted derivatives, have been described by Brühlmann, C., et al., in *J. Med. Chem.* 2001, 44, 3195-3198.

Kang, S.Y., et al., describe in *J. Nat. Prod.,* 2001, 64, 683-685, coumarins isolated from Angelica gigas which inhibit acetylcholinesterase. In this study it was found that cyclized coumarin derivatives such as dihydropyranocoumarins and furanocoumarins showed the best inhibitory activity compared to simple coumarins lacking a cyclized isoprenyl unit. One of these coumarins, decursinol, which shows a high inhibitory activity towards to acetylcholinesterase in vitro, has been recently tested *in vivo*, with respect to β-amyloid peptide-induced memory impairment (Yang, J., et al., *Progress in Neuro-Psychopharmocology and Biological Psychiatry* 28 (2004), 25-30). It could be shown that pretreatment of mice with decursinol for four weeks significantly attenuated the β-amyloid peptide 1-42 induced impairment in passive avoidance performance. Decursinol also blunted the β-amyloid peptide 1-42 induced decrease in alternation behaviour in the Y-maze test without change in locomotor activity.

Miazawa, M., et al., describe in Natural Products Letters, 15 (3), 205-210 (2001) the inhibition of acetylcholinesterase activity of *inter alia* auraptene, a coumarin derivative, isolated from *Citrus paradisi.*

Furthermore, the use of coumarin derivatives as lipid-rich plaque inhibitors and ACAT inhibitors is described in WO 03/059900 and the use of various coumarin derivatives as microtubule stabilizing agents and their therapeutic use is described in WO 02/062293.

Despite the substantial progress, there is still a great interest in finding better AChE inhibitors to give faster penetration in the CNS, longer duration of action and lower toxicity. Therefore, it is the object of the present invention to provide acetylcholinesterase inhibitors which can give faster penetration in the CNS, longer duration of action and lower toxicity in order to provide efficient means of treating disorders, in particular neurological and neuromuscular disorders, involving low levels of acetylcholine in the brain.

This object has been achieved with the use of a coumarin derivative having a coumarin skeleton of formula I 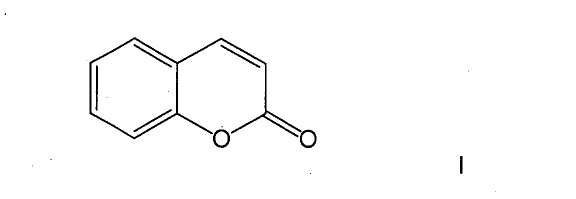 wherein the coumarin skeleton is substituted by one OR¹ and one OR²
whereby
R¹ is C₁ to C₆ alkyl, C₃ to C₇ cycloalkyl or C₂ to C₆ alkenyl and
R² is hydrogen or a sugar moiety,
or a pharmaceutically acceptable salt thereof,
for the preparation of a medicament for the prophylaxis or treatment of disorders which involve low levels of acetylcholine in the brain.

Surprisingly, it was found that the above coumarin derivatives of formula I inhibit acetylcholinesterase *in vitro* and enhance acetylcholine concentration in the brain *in vivo*. Therefore, these coumarin derivatives can ameliorate disorders connected with deficits in learning and memory functions.

### Brief Description of the Figures

Figure 1 shows the inhibitory effect of different concentrations of GNT, SCT and SCN on AChE.
Figure 2 shows the time dependent inhibitory effect of different concentrations of GNT, SCT and SCN in comparison with medium control.
Figure 3 shows the effect of the vehicle on the extracellular ACh concentration in the nucleus accumbens, whereby the arrow indicates the time when the i.c.v. injection was started.
Figure 4 shows the effects on GNT at different doses with respect to the increase of the extracellular ACh concentration.
Figure 5 shows the effects of SCT and SCN with respect to the increase of the extracellular ACh concentration.
Figure 6 shows the effect of SCT in the object recognition task on the scopolamine-induced learning impairment.
Figure 7 shows the effect of SCT on spontaneous alternation in the T-maze on the scopolamine-induced memory impairment.
Figure 8 shows the effect of SCT on open field locomotor behaviour on move time and move distance in the open field paradigm.

The coumarin derivative of formula I used in accordance with the present invention will be described in more detail below.

R¹ is as described above, preferably C₁ to C₆ alkyl or C₂ to C₆ alkenyl, more preferably C₁ to C₄ alkyl, most preferably methyl or ethyl.

In the following, the definitions for substitutent R¹ in formula I are described in more detail.

Alkyl is straight chain or branched alkyl having 1 to 6 carbon atoms, such as methyl, ethyl, n-propyl, isopropyl, n-butyl, isobutyl, tert-butyl, n-pentyl, isopentyl, neopentyl, or hexyl, more preferably 1 to 4 carbon atoms. Alkyl may be substituted or unsubstituted.

Cycloalkyl is an alkyl ring having 3 to 7 carbon atoms, such as cyclopropyl, cyclobutyl, cyclopentyl, cyclohexyl, or cycloheptyl, more preferably 3 to 6 carbon atoms. Cycloalkyl may be substituted or unsubstituted.

Alkenyl is straight chain or branched alkenyl having 2 to 6 carbon atoms such as vinyl, allyl, methallyl, buten-2-yl, buten-3-yl, penten-2-yl, penten-3-yl, penten-4-yl, 3-methyl-but-3-enyl, 2-methyl-but-3-enyl, 1-methyl-but-3-enyl, or hexenyl, preferably 2 to 4 atoms. Alkenyl may be substituted or unsubstituted.

Suitable substitutents include OH, halogen selected from F, Cl, Br and I, NH₂, NO₂, CN or CF₃.

R² is as defined above. The sugar moiety can be any suitable sugar known in the art, preferably a monosaccharide, whereby a pyranosidic (6-membered ring) or furanosidic (5-membered ring) sugar residue which can be modified are preferred. Examples of suitable sugar residues include:
(4-hydroxyphenyl)acetyl]-β-D-glucopyranosyl]
α-D-glucopyranosyl
β-D-glucopyranosyl
(6-deoxy-α-L-mannopyranosyl)
[3,4,6-tri-O-acetyl-2-(acetylamino)-2-deoxy-β-D-glucopyranosyl]
6-O-(6-deoxy-α-L-mannopyranosyl)-β-D-glucopyranosyl]
(6-O-acetyl-β-D-glucopyranosyl)
(6-O-D-apio-β-D-furanosyl-β-D-glucopyranosyl)
[2,3,4-tri-O-acetyl-6-O-(2,3,5-tri-O-acetyl-D-apio-β-D-furanosyl)-β-D-glucopyranosyl]
(2,3,4,6-tetra-O-acetyl-β-D-glucopyranosyl)
(O-α-L-arabinbpyranosyl-(1→3)-O-β-D-galactopyranosyl-(1→6)-β-D-galactopyranosyl)
(O-α-D-glucopyranosyl-(1→4)-O-α-D-glucopyranosyl-(1→4)-α-D-glucopyranosyl)
(2-O-acetyl-β-D-glucopyranosyl)
(3,6-di-O-acetyl-β-D-glucopyranosyl)
(2-O-D-apio-β-D-furanosyl-β-D-glucopyranosyl)
[2-O-(6-deoxy-α-L-mannopyranosyl)-β-D-glucopyranosyl]
(O-2,3,4-tri-O-acetyl-6-deoxy-α-L-mannopyranosyl-(1→2)-O-[2,3,4-tri-O-acetyl-6-deoxy-α-L-mannopyranosyl-(1→6)]-3,4-di-O-acetyl-β-D-glucopyranosyl)
[2-O-acetyl-6-O-(6-deoxy-α-L-mannopyranosyl)-β-D-glucopyranosyl]
[6-O-(6-deoxy-α-L-mannopyranosyl)-β-D-glucopyranosyl]
(2,6-di-O-acetyl-β-D-glucopyranosyl)
[2-O-(4-O-acetyl-6-deoxy-α-L-mannopyranosyl)-β-D*-glucopyranosyl]
β-D-xylofuranosyl
[3,4,6-tri-O-acetyl-2-O-(2,3,4-tri-O-acetyl-6-deoxy-α-L-mannopyranosyl)-β-D-glucopyranosyl]
(6-O-β-D-glucopyranosyl-β-D-glucopyranosyl) = β-D-gentiobiosyl β-gentiobiosyl hexaacetate
[2-(acetylamino)-2-deoxy-β-D-glucopyranosyl]
[3,4,6-tri-O-acetyl-2-(acetylamino)-2-deoxy-β-D-glucopyranosyl]
(4-O-acetyl-β-D-glucopyranosyl)
[2-(acetylamino)-2-deoxy-4,6-O-(phenylmethylene)-β-D-glucopyranosyl]
β-D-Glucopyranosiduronyl
[4,6-O-[(3,4-dihydroxyphenyl)methylene]-β-D-glucopyranosyl]
(4,6-O-cyclohexylidene-β-D-glucopyranosyl)
[4,6-O-(1-methylethylidene)-β-D-glucopyranosyl].

Most preferably, the sugar moiety is D-glucopyranose or a modified derivative thereof, most preferably D-glucopyranose. The sugar moiety can be linked to the molecule via α- or β- linkage, preferably a β-linkage. Preferably, R² is hydrogen or β-D-glucopyranose, more preferably hydrogen.

The substitution may be present on any position on the coumarin skeleton. Preferably, the coumarin derivative can be represented by the following formula I(a): 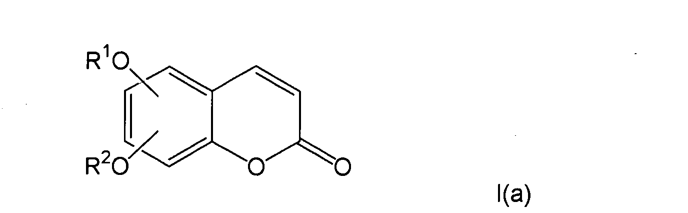 whereby OR¹ and OR² are as defined above. More preferably, either OR¹ or OR² can be present in position 6 or 7 and it is even more preferred that one of OR¹ and OR² is present in position 6 and the other in position 7. Most preferably, the coumarin derivative can be represented by the following formula I(b): 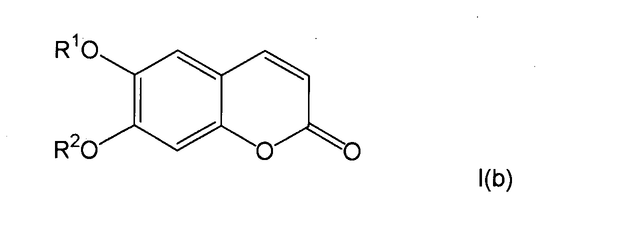 whereby OR¹ and OR² are as defined above.

Preferred examples of the coumarin derivative of formula I are scopoletin (SCT, 1) and its glycoside scopolin (SCN, 2).

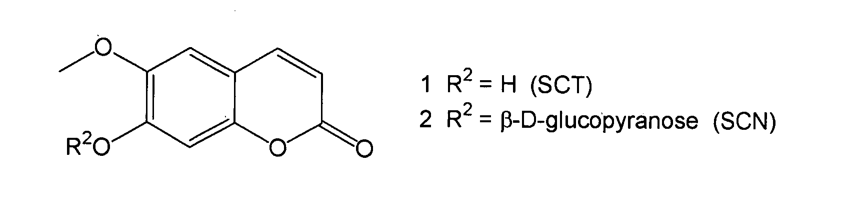

Depending on the nature of R¹ and R², the coumarin derivatives of formula I may be present in the form of pharmaceutically acceptable salts of these.

The term "pharmaceutically acceptable salts" refers to salts prepared from pharmaceutically acceptable non-toxic bases or acids, including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines and cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyarnine resins, procaine, purines, theobromine, triethylamine, trimethylamine, tripropylamine, tromethamine and the like.

When the coumarin derivative of the formula I is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the coumarin derivative of formula (I) are meant to also include the pharmaceutically acceptable salts thereof.

Coumarin derivatives of formula I are inhibitors of acetylcholinesterase and can enhance the brain acetylcholine concentration and as such are useful in the treatment of disorders which involve low levels of acetylcholine in the brain. In this context, the term "treatment" is meant to include the prophylaxis as well as the treatment of these disorders including ameliorating the effects of these disorders. Specifically, such disorders include neurological and neuromuscular disorders, more specifically, disorders selected from the group, consisting of Alzheimer's disease, senile dementia, ataxia, myasthenia gravis and Parkinson's disease. Specifically, the treatment of Alzheimer's disease is indicated.

Any suitable route of administration may be employed for providing a mammal, especially a human, with an effective dosage of a coumarin derivative of formula I. For example, oral, rectal, topical, parenteral, including subcutaneous, intravenous and intramuscular, ocular, pulmonary, nasal and the like, may be employed. Dosage forms include tablets, troches, dispersions, suspensions, solutions, capsules, creams, ointments, aerosols and the like. Preferably the coumarin derivatives of formula I are administered orally or topically.

The effective dosage of an active ingredient employed may vary depending on the particular compound employed, the mode of administration, the condition being treated and the severity of the condition being treated. Such dosage may be ascertained readily by a person skilled in the art.

When treating Alzheimer's disease, as well as other diseases or disorders for which coumarin derivatives of formula I are useful, generally satisfactory results are obtained when the coumarin derivatives are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

The coumarin derivative of formula I is preferably formulated into a dosage form prior to administration. Such a dosage form typically includes a pharmaceutical composition comprising a coumarin derivative of formula I and a suitable pharmaceutical carrier.

Such pharmaceutical compositions are prepared by known procedures using well-known and readily available ingredients. In making these formulations, the active ingredient (a coumarin derivative of formula I) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl, propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to.the patient.

The coumarin derivatives of formula I can be obtained either from natural sources or can be synthetically prepared. Some coumarin derivatives of formula I may be commercially available.

For example, SCN and SCT can be isolated from *Scopolia carniolica*, as described, e.g., in more detail in the examples.

Furthermore, the coumarin derivatives of formula I can be prepared by appropriately derivatizing the coumarin structure as is well known to the person skilled in the art. For example, the methods as described in Glaser; Arch.Pharm.(Weinheim Ger.); 1928; 575, Chaudhury et al.; J.Chem.Soc.; 1948; 1671, Seka; Kallir; Chem.Ber.; 64; 1931; 909, 915, Cingolani; Gaudiano; Rend. Ist. super. Sanita; 19; 1956; 1256, 1260, Aghoramurthy; Seshadri; J.Sci.Ind.Res.; 11 B; 1952; 411., Eijkman; Recl.Trav.Chim.Pays-Bas; 3; 1884; 191, Kuwajima, Hiroshi; Morita, Masami; Takaishi, Kiyokazu; Inoue, Kenichiro; Fujita, Tetsuro; et al.; Phytochemistry; 31; 4; 1992; 1277-1280, Yuldashev, M. P.; Chem.Nat.Compd.(Engl.Transl.); 38; 2; 2002; 192 - 193; Khim.Prir.Soedin.; 2; 2002; 159 - 160, Tsukamoto, Hiroki; Hisada, Sueo; Nishibe, Sansei; Chem.Pharm.Bull.; 33; 1; 1985; 396-399, Lumonadio Luyengi; Vanhaelen, Maurice; Phytochemistry; 24; 10; 1985; 2387-2390, Kuroyanagi, Masanori; Shiotsu, Michiko; Ebihara, Tsuyoshi; Kawai, Hideaki; Ueno, Akari; Fukushima, Seigo; Chem.Pharm.Bull.; 34; 10; 1986; 4012-4017, Hauer, Hermann; Ritter, Thomas; Grotemeier, Gregor; Arch.Pharm.(Weinheim Ger.); 328; 10; 1995; 737-738, Demyttenaere, Jan; Syngel, Kris Van; Markusse, A. Peter; Vervisch, Stijn; Debenedetti, Silvia; Kimpe, Norbert; Tetrahedron; 58; 11; 2002; 2163 - 2166, Batirov, E. Kh.; Matkarimov, A. D.; Malikov, V. M.; Seitmuratov, E.; Chem.Nat.Compd.(Engl.Transl.); 18; 6; 1982; 654-657; Khim.Prir.Soedin.; 18; 6; 1982; 691-695, Yuldashev, M. P.; Batirov, E. Kh.; Malikov, V. M.; Chem.Nat.Compd.(Engl.Transl.); 16; 2; 1980; 125-128; Khim.Prir.Soedin.; 16; 2; 1980; 168-172, Zeile; Heusner; Chem.Ber.; 90; 1957; 2800, 2805 or Fodor; Kovacs; J.Chem.Soc.; 1953; 2341 or suitable modifications thereof can be followed.

The following examples have been included to further illustrate the present invention and are not intended to limit the scope of the claims.

### EXAMPLES

### 1. Study of in vitro and in vivo AChE inhibitory effect of SCT and SCN

### 1.1 Experimental

### 1.1.1 Extraction and Isolation

The rhizomes of *Scopolia carniolica* (202 g) were crushed to coarse powder and extracted with 600 mL methanol for 10 h in a Soxhlet extractor. Upon removal of solvent in vacuo, the methanolic extract yielded 32 g. This crude extract was suspended in CH₂Cl₂ and fractionated by silica gel flash column chromatography (5x35 cm) using a step-gradient of CH₂Cl₂-MeOH (CH₂Cl₂; CH₂Cl₂-MeOH 99:1, 98:2, 95:5, 90:10, 75:25, 60:40, 35:65; MeOH; 500 ml each) to give fourteen fractions (A 1-14). Fractions A 5/6 (650 mg) and fraction A 10 (300 mg) contained fluorescent spots at 366 nm on silica gel TLC plates 60F₂₅₄ (Merck, Darmstadt, Germany, developed in ethyl acetate/acetic acid anhydride/formic acid/water, 100:11:11:260) indicating a coumarin content. Both fractions were further purified by Sephadex CC (2x100 cm) with methanol to yield 51 mg SCT and 87 mg SCN, respectively. SCT and SCN were obtained from methanol as whitish needles and white microcrystalline powder, respectively.

### 1.1.2 TLC- and Microplate Test

The AChE inhibitory activity of the methanolic crude extract was qualitatively determined in a TLC assay using Ellman's method (Ellman, et al, 1961). Additionally a test to exclude false positive results was conducted described by Rhee et al, 2003. The used silica gel TLC plates 60F₂₅₄ (0.2 mm thickness) were purchased from Merck (Darmstadt, Germany). 10 µL of the crude extract dissolved in methanol (10 mg/mL) and, as reference substance, 10 µL of a solution of 100 µg and 10 µg galathamine hydrobromide (GNT, purchased from Tocris; Cookson Ltd, Avonmounth UK Bristol), respectively, were spotted on the TLC plate and developed in the solvent toluene/ether/methanol (1:1:2). Enzyme inhibitory activities were detected by spraying the substrate, dye and enzyme described by Rhee and coauthors (Rhee et al, 2001).

SCT, SCN, and GNT were quantitatively tested applying a modified Ellman's method in a 96-well microplate assay described by Ingkaninan et al, 2000. In both test systems, the enzyme (acetylcholinesterase, Sigma-Aldrich Chemie GmbH, Steinheim, Germany) hydrolyzes the substrate acetylthiocholine (acetylthiocholiniodid, Sigma-Aldrich Chemie GmbH, Steinheim, Germany) to produce thiocholine. This reacts with Ellman's reagent (5,5'-dithiobis-(2-nitrobenzoic acid), Sigma-Aldrich Chemie GmbH, Steinheim, Germany) resulting in 5-thio-2-nitrobenzoate and the yellow colored 2-nitrobenzoate-5-mercaptothiocholine. This latter was visually detected in the TLC test.

In the microplate assay the absorbance of 2-nitrobenzoate-5-mercaptothiocholine was quantitatively measured at 405 nm with a Chameleon 5025 (Hidex, Finland) microplate reader 0, 15, 30, 45, and 60 minutes after adding 25 µL of the 0.22 U/mL AChE to the reaction mixture. The self absorbance of all ingredients of the assay was corrected by subtracting the absorbance at time zero from the subsequent absorbance values. For the microplate assay, SCT and SCN were dissolved in DMSO to stock solutions of 100, 50, 25, 12.5, 6.25 mM and diluted with a buffer (50 mM Tris-HCl, pH 8) at 1:100; stock solutions of GNT in DMSO (10, 2.5, 0.625, 0.156, and 0.039 mM) were diluted with the buffer to give 100, 25, 6.25, 1.56, and 0.39 µM. Final concentration of DMSO in the microplate assay was 1%, to ensure it did not disturb the test system. For all three substances the concentration dependence of their AChE inhibitory activity was determined. Percentage of the enzyme inhibition was calculated by comparing the rates for the sample to the blank (containing 1% DMSO) and analyzed with Student's t-test. For statistical processing the SPSS 11.5 program package was used.

### 1.1.3 In vivo study

**Surgery and push-pull technique:** Sprague-Dawley rats (250-350 g) were anaesthetized with urethane 1,2 g/kg, i.p. The head was fixed in a stereotaxic frame and a push-pull cannula (outer tubing: o.d. 0.75 mm, i.d. 0.41 mm; inner tubing: o.d. 0.26 mm, i.d. 0.13 mm) was mounted on a microdrive. This was stereotaxically inserted through a hole in the skull into the nucleus accumbens (coordinates, mm from bregma: AP, +1.2; L, +2.5; V, -7.8; Paxinos & Watson, 1998). A second hole was drilled into the skull for the i.c.v. application of drugs (coordinates, mm from bregma: AP, -2,8; L, +1,5) The nucleus accumbens was superfused at 20µL/min with artificial cerebrospinal fluid (aCSF), pH 7.2. The CSF consisted of: NaCl,140; KCI, 3; CaCl₂, 1.2; MgCl₂ 1; Na₂HPO₄, 1; NaH₂PO₄, 0.3; glucose 3.0 (mmol I⁻¹), neostigmine 1.0 (µmol/l⁻¹). After 1 h of the cannula insertion the samples were collected into tubes kept at -20° C in time periods of 10 min and then stored at -80° C until acetylcholine (Ach) was determined.

**Application of drugs:** Drugs were dissolved in 20 µL of DMSO (GNT) or DMSO:EtOH 1:1 (coumarins). Using a motorized microsyringe connected to a stereotaxic microdrive, the tip was introduced with an angle of 60° through the drug application-hole into the ipsilateral ventricle (coordinates mm from bregma: AP, -0,9; L, +1,5; V, -3,8) via a cannula (o.d., 0,65; i.d., 0,38). The drug solutions were injected at 2µL/min. The control rats were injected with the vehicle (DMSO: EtOH 1:1).

**Histology**: At the end of the experiment the rat was killed with an overdose of urethane, the brain was removed and stored in buffered formaldehyde solution (7%). Serial coronal sections were cut at 50 µm intervals. Localisation of the push-pull cannula and i.c.v. microinjection was verified. Experiments with unsatisfactory localisation were discarded.

**Determination of acetylcholine:** (Ach): ACh content in the samples was determined by HPLC with a cation-exchanging analytical column and a post-column reactor followed by electrochemical detection (Damsma et al, 1997; Prast et al; 1998). The system consisted of a HPLC-pump (JASCO PU-1580, Jasco Corporation, Tokyo, Japan), a pulse dampener and an autosampler (AS-4000, Hitachi Ltd., Tokyo, Japan) with a 100-µL sample loop. The mobile phase was (mm): K₂HPO₄, 100; KCI, 5; tetramethylammonium hydroxide, 1; Na-EDTA, 0.1 and 0.5 mL/L Kathon® CG (Rohm & Haas, Frankfurt/Main, Germany), pH adjusted to 7.9 with H₃PO₄, pumped at a flow rate of 0.4 mL/min. ACh was separated on an analytical column (80 x 3 mm, Kromasil® 100-5 C18) loaded with laurylsulphate. At the post-column enzyme reactor (20 x 1 mm Kromasil® 100-10 NH₂) to which AChE (Sigma-Aldrich Chemie, Steinheim, Germany) and choline oxidase (Sigma-Aldrich Chemie, Steinheim, Germany) were bound, ACh was hydrolyzed to acetate and choline. Subsequently, choline was oxidized to betaine and hydrogen peroxide. The peroxide was electrochemically detected by a UniJet platin electrode (3mm) at +500 mV with an amperometric detector (BAS LC-4, Bioanalytical Systems, West Lafayette, IN, USA). The detection limit for ACh (signal to noise ratio = 3) was 10 fmol per sample. ACh was quantified with external standards that were injected at the beginning and the end of the analysis.

Statistical significance of drug effects on the extracellular ACh concentration was calculated by Friedman's analysis of variance, followed by Wilcoxon's signed rank test for paired data (a p<0.08, * p<0.05, ** p<0,001) using the mean concentration rate before the administration of drugs as controls. Data are shown as means ± SEM of relative values. The mean concentration rate before drug administration was taken as 1.0.

### 1.2 Results

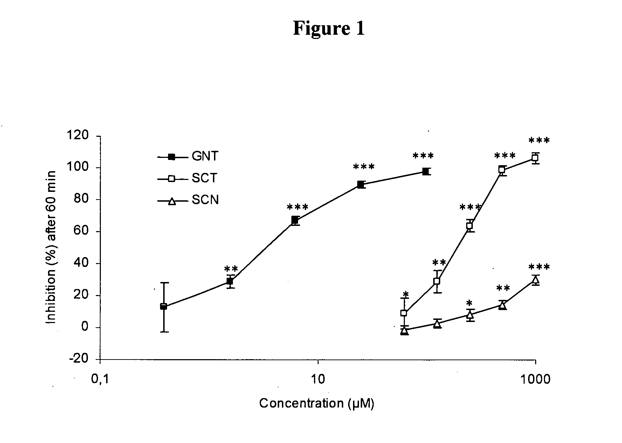

According to the solubility of the comprising coumarins, a methanolic extract of the rhizomes of *S. carniolica* was produced and screened in a bioautographic TLC test in order to evaluate its AChE inhibitory activity. The fluorescent TLC spots on Rf 0.6 and 0.4, corresponding to SCT and SCN, respectively, emerged as positive. False positive results could be excluded by applying a TLC bioautographic assay as described by Rhee et al, 2003. Using different chromatographic methods, SCT and SCN were isolated and identified by TLC (Wagner et al, 1983), ¹H-NMR (Nowak et al, 2002) and LC-MS (Ganzera et al, 1997). The activity of the isolated compounds was quantitatively tested and compared to GNT in a microplate assay (Ingkaninan et al, 2000). All three drugs inhibited AChE activity in a dose dependent manner (Fig. 1).

Figure 1: Microplate assay: Inhibitory effect of different concentrations of GNT, SCT, and SCN on AChE. Statistical analysis: data are means ± SD; *** p<0.001, ** p<0.01, * p<0.05, Student's test of absorption data after 60 min in comparison with medium control, n=4.

The concentration of SCT required for half-maximal AChE inhibition (IC₅₀) was determined to be 200.1 µM (Cl₉₅ 183.0 - 217.2). The IC₅₀ obtained for GNT fits well to the data already described in literature, e.g. Lopez et al. (2002). They report an IC₅₀ of 1.07 µM, using pre-incubation of the enzyme with GNT before adding Ellman's reagent. Rhee and coauthors (2001) report an activity of about 50 % at 2.5 µM. In our assay GNT exhibited an IC₅₀ of 4.2 µM (Cl₉₅ 3.8 - 4.6). SCN, however, exerted only an AChE inhibitory activity of 30.1% at 1000 µM (p<0.001, measurement after 60 min). Although the inhibition for 1000, 500, and 250 µM proved to be significant, the IC₅₀ of SCN was not determined.

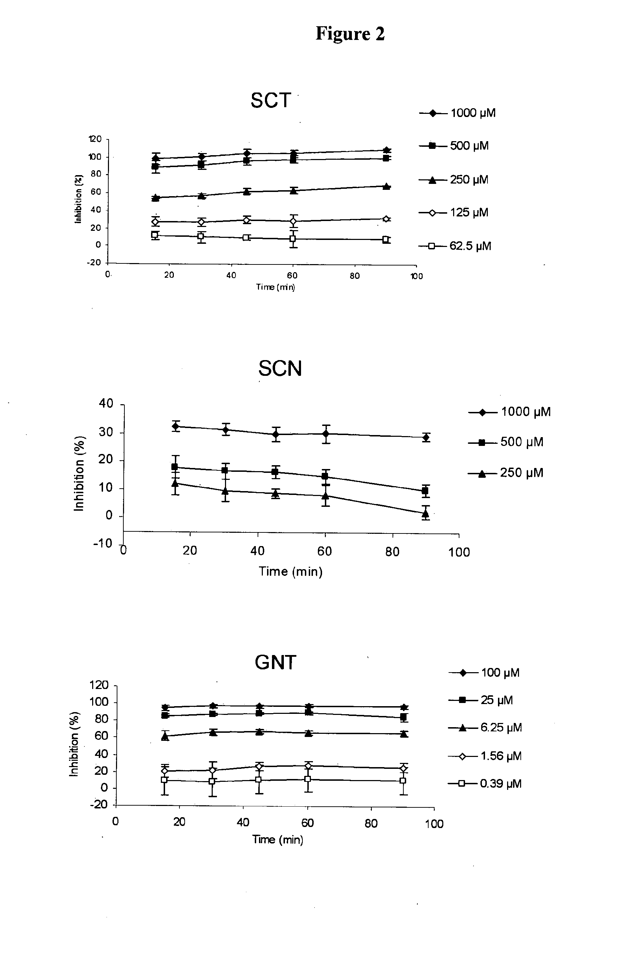

The *in vitro* inhibition of AChE caused by five different concentrations of SCT was measured over 90 min. The data show (Fig. 2) that at lower concentrations (62.5, 125 µM) the level of inhibitory activity is reached within the first 15 min of adding the enzyme to the substrate and lasts for at least 90 min. At higher concentrations of SCT (250, 500, 1000 µM) a slight, almost insignificant increase in inhibitory activity is determined up to 90 min. In the case of SCN the inhibitory activities of the three highest concentrations (Fig. 2) reach their maximum extent within the first 15 min followed by a slight decrease towards the endpoint of measurement at 90 min. However, the inhibitory activity of SCN is 5.4 x 10² lower compared to GNT (calculated for 30% inhibition, measured after 60 min). The extent of AChE inhibition caused by GNT is constant within the time of measurement (Fig. 2), indicating a competitive manner of inhibition as already described by Bores et al, 1996.

Figure 2: Microplate assay: Time dependent inhibitory effect of different concentrations of SCT, SCN, and GNT in comparison with medium control. Data are means ± SD, n=4.

**In vivo extracellular acetylcholine (ACh) concentration**: A further important goal was to test the compounds for their effectiveness in enhancing cholinergic transmission in the brain *in vivo*. For this purpose, we monitored extracellular ACh concentration in the brain of rats with the push-pull technique and injected the putative inhibitors into the cerebral ventricle. In analogy to the *in vitro* experiments, the effect of GNT was as well observed in the *in vivo* study. Its effect on ACh in rat brain has not yet been comprehensively investigated.

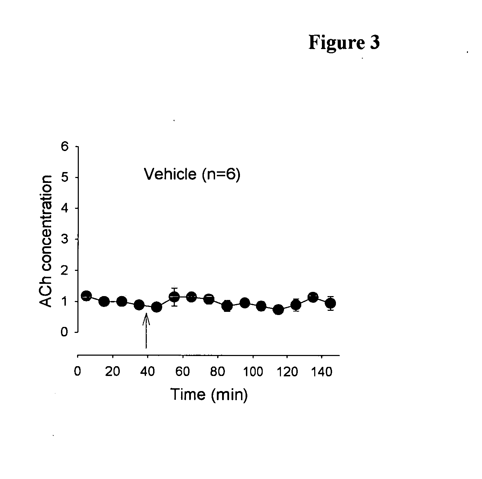

**Basal concentration and injection of vehicle**: 60min after beginning the superfusion, the extracellular ACh concentration rate reached a stable level, which did not change throughout the experiment. The basal concentration of ACh in the nucleus accumbens of urethane-anaesthetized rat was 83,30 ± 10,82 fmol/min (n=39). The i.c.v. injection of vehicles DMSO:EtOH 1:1 (Fig. 3) and DMSO (not shown) did not significantly influence the basal concentration of ACh.

Figure 3: Effect of vehicle (DMSO:EtOH; 1:1) on the extracellular concentration of ACh in the nucleus accumbens. Arrows indicate time when i.c.v. injection (20 µl at 2 µl/min) was started. Data are relative values (means ± SEM). Four samples before start of the injection were taken as controls.

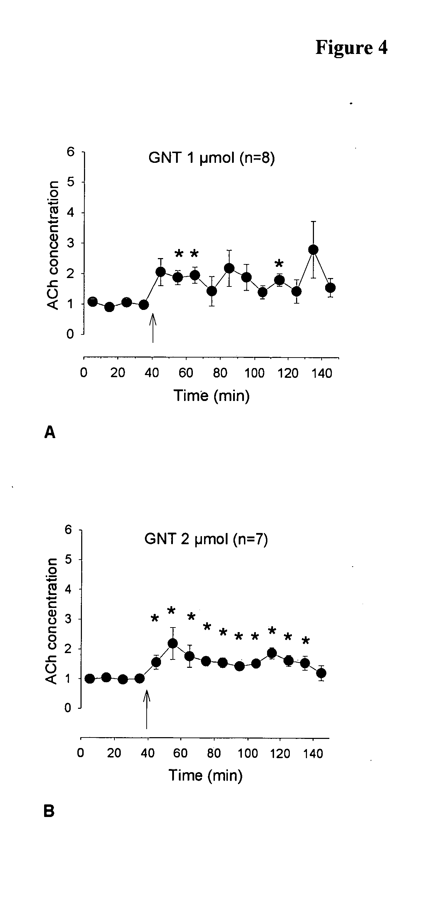

Administration of 1 and 2 µmol GNT induced an increase in the extracellular concentration of ACh. Both doses caused an effect of similar magnitude (means ± SEM: 1,78 ± 0,32 and 1,68 ± 0,20). Though, in the group treated with the lower dose the effect of GNT showed a higher variability (Fig. 4).

Figure 4: Effects of GNT A: 1 µmol and B: 2 µmol on the extracellular concentration of ACh in the nucleus accumbens. Arrows indicate time when i.c.v. injection (20 µL at 2 µL/min) of GNT was started. Data are shown as relative values (means ± SEM). Four samples before injection were taken as controls. * p<0,05, baseline samples vs. the post-injection samples.

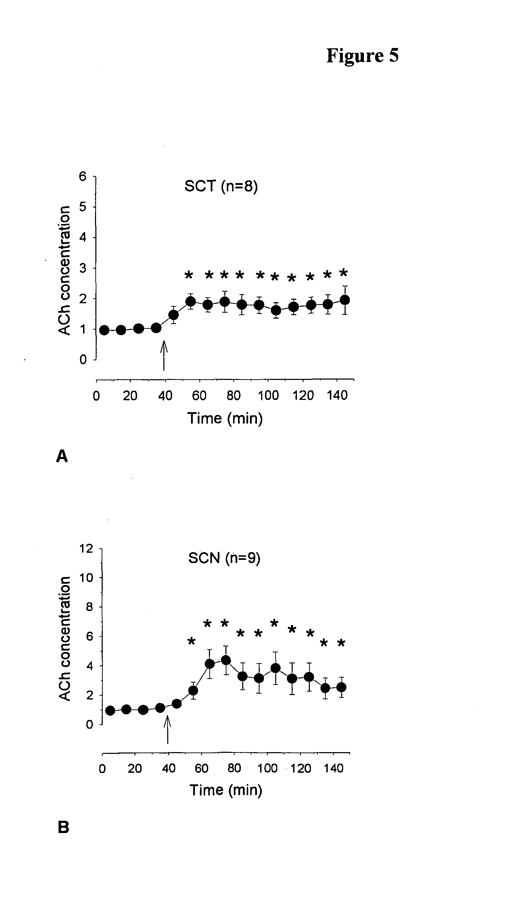

Both coumarins induced a pronounced and long-lasting increase in the extracellular concentration of ACh. The injection of SCT enhanced the concentration of ACh to a continuous level of about 1.7 times the basal value (mean ± SEM: 1,74 ± 0,24) (Fig. 5A).

Figure 5: Effects of A: SCT (2 µmol) and B: SCN (2 µmol) on the extracellular concentration of ACh in the nucleus accumbens. Arrows indicate time when i.c.v. injection (20 µl at 2 µl/min) was started. Data are relative values (means ± SEM). The four samples before the injection were taken as controls. ^{a} p<0,08;* p<0,05, baseline vs. post-injection samples.

In contrast to the results obtained from the *in vitro* experiments, the increase in ACh by the glucoside SCN was higher (mean concentration after drug application 3.18 ± 0.78) and showed an initial peak concentration of about 400% of basal value followed by a decline to about 200 %. This phenomenon may be associated with the slight decrease of the AChE inhibitory activity, which was also manifested in the enzyme assay of SCN (Fig. 2). Alternatively it may be a result of the metabolism of the coumarin itself or of a down-regulation of the ACh synthesis.

### 2. Study of learning and memory improving capacity of SCT

### 2.1. Materials and methods

### 2.1.1 Animals

C56BL/6N female, 3-month mice, weighing 20 - 27 g, were used in the present study. The animals were housed in groups of six to ten in cages (40 (L) x 25 (W) x 15 (H) cm) placed in a room at a constant temperature (22±1 °C), 12-light/ 12-dark cycle (07:00-19:00 h), with food and water *ad libitum*. Experiments were conducted between 09:00 and 16:00h.

### 2.1.2 Surgery

Mice were anaesthetized with Pentobarbital sodium (60 mg/kg, i.p.) and placed in a stereotaxic frame. A guide cannula (gauge 25, o.d. 0.52 mm, i.d, 0.26 mm and 7 mm long) was stereotaxically implanted through a hole on the skull into the brain area 0.5 mm higher than the third cerebral ventricle (-0.1 mm posterior, 0.8 mm lateral to bregma, -2.2 mm ventral to the dura) (Franklin and Paxinos, 1997). Guide cannula was secured to the skull using two small screws and dental cement. The microinjector (Hamilton gauge 32, o.d. 0.24 mm and i.d. 0.11 mm, 7.5 mm long) protruded 0.5 mm from the tip of the guide cannula to reach the cerebral ventricle. Mice recovered for 5 days before any experimentation was done.

### 2.1.3 Drugs

Scopolamine hydrobromide (Sigma, St. Louis, MO, U.S.A.) was dissolved in saline. SCT (Sigma-Aldrich Chemie GmbH, Steinheim, Germany) was dissolved in vehicle solution (DMSO:sterile water, 3:7). The animals were randomly divided into experimental groups and administered a cerebral ventricle injection of saline, scopolamine, SCT or scopolamine and SCT in a volume of 1 or 2 µl during 1 min. The injector was left in place an extra 1 min for drug diffusion after injection. These substances and vehicle were injected 15 min prior to the test.

### 2.1.4 Experimental design and drug treatment

Animals were randomly divided into three experimental groups (6 mice per group) as follows: vehicle, scopolamine hybrobromide 20 µg (50 nmol) in 1µl ICV, SCT 2 µg (10 nmol) in 2µl ICV and scopolamine hybrobromide 20 µg plus SCT 2 µg ICV. All substances were injected 15 min before the test (T-maze and open field) or before the training trial T1 (object recognition test) through the 7.5 mm long injector connected with i.d. 0.28 mm polyethylene tubing to 5 µl Hamilton syringe.

### 2.1.5 Object recognition task experiments

### Apparatus

The test apparatus consisted of a box made of Plexiglas (40 (L) x 25 (W) x 15 (H) cm), which was illuminated by a lamp above the box. In the different parts of the apparatus, the light intensity was equal at 100 lux. The objects to be discriminated were made of plastic or metal, were in the different shapes: cubes and cylinders 1.5-cm high in white or green colour; they could not be displaced by mice. In addition, these objects had no genuine significance for mice and had never been associated with reinforcement.

### Procedure

The object recognition test was performed as described elsewhere (Ennaceur and Delacour, 1988). On the day before testing, the animals were allowed to explore the apparatus for 10 min (without objects), while on the testing day, a session of two 10-min trials was given. During the training trial (sample trail) (T1), two identical objects were placed opposite sides 8 cm from the short walls. A mouse was placed in the middle of apparatus and was left to explore these two identical objects in 10 min. After T1, the mouse was put back in its home cage and a 24- hour inter-trial interval was given. Subsequently, the testing trial (choice trial) (T2), was performed in 10 min. During T2, a novel object *(N)* replaced one of the samples presented in T1, hence, the mouse was now exposed to two different objects: the familiar *(F)* and the novel one *(N)*. All combinations and locations of objects were used in a balanced manner to reduce potential biases due to preferences for particular locations or objects. To avoid the presence of olfactory trails, the apparatus and the objects were thoroughly cleaned after each trial.

Exploration was defined as follows: directing the nose toward the object at a distance of less than 0.5 cm and/or touching the object with the nose. Turning around or sitting on the object was not considered as exploratory behavior. The times spent by mice in exploring each object during T1 and T2 were recorded manually by using a stopwatch. From this measure, a series of variables were then calculated: the total time spent in exploring the two identical objects in T1, and that spent in exploring the two different objects, familiar and novel in T2. The discrimination between the familiar and the novel object during T2 was measured by comparing the time spent in exploring the familiar sample with that spent in exploring the new object. As this time may be biased by differences in overall levels of exploration, a discrimination index *(D)* was then calculated; *D=N-F*/*N+F. D* is the discrimination ratio and represents the difference in exploration time expressed as a proportion of the total time spent exploring the two objects in T2.

### 2.1.6 T-maze continuous alternation experiments

### Apparatus

The T-maze was constructed according to the measures provided by Gerlai (Gerlai, 1998). The apparatus was made of grey Plexiglas. The guillotine doors could be operated by the experimenter.

### Procedure

Testing of a mouse consisted of one single session, which started with 1 forced-choice trial, followed by 14 free-choice trials.

### Forced-choice trial

In the first trial, the 'forced-choice trial', either the left or right goal arm is blocked by lowering the guillotine door. After the mouse has been released from the start arm, it will negotiate the maze, eventually enter the open goal arm, and return to the start position. There, the animal will be confined for 5 s by lowering the guillotine door of the start arm.

### Free-choice trials

During 14 'free-choice' trials, the mouse can choose freely between the left and right goal arm. After opening the guillotine door of the start arm the animal is free to choose between both goal arms (all guillotine doors open). As soon as the mouse has entered one goal arm, the other goal arm is closed. The mouse eventually returns to the start arm, and the next free-choice trial starts after a 5-s confinement in the start arm. A session is terminated and the animal is removed from the maze as soon as 14 free-choice trials have been performed or 30 min have elapsed, whatever event occurs first. During the session, the animals are never handled by the experimenter. In order to isolate the cognitive component, care was taken to diminish the odour cues present by cleaning the maze with a damp sponge before each new animal started its session, as these cues are susceptible of influencing the alternation rate.

### 2.1.7 Open field experiments

The open field consisted of a transparent plastic box with white floor (41 x 41 x 41 cm) equipped with an automated activity monitoring system (True Scan, Coulbourn Instruments, Allentown, USA). Illumination at floor level was 150 lux. The area of the open field was divided into a 28 x 28 cm central zone and the surrounding border zone. Mice were individually placed into the centre of the open field and their behaviours were tracked with the activity monitoring system. The time spent in box and the overall distance travelled by the mice were monitored for 30 min. (Prut and Belzung, 2003).

### 2.2 Results

At the doses tested, the mice showed normal behaviour. Cholinergic symptoms such as salivation, lacrimation, and diarrhea were not observed.

### 2.2.1 Effect of SCT on scopolamine-induced learning-deficit in object recognition.

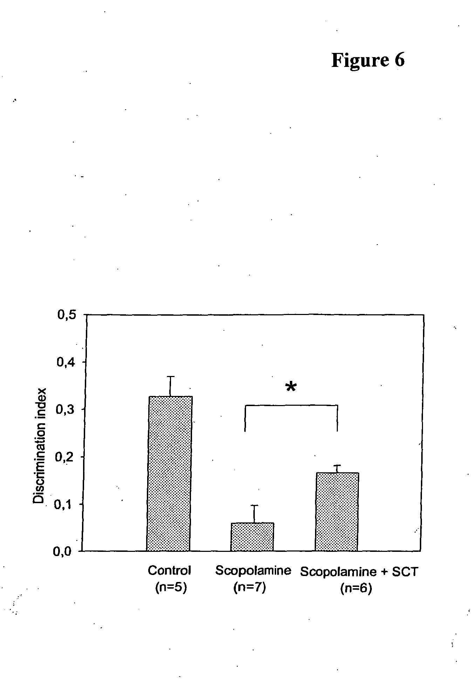

Following habituation to the arena two identical objects were presented to the mouse during TI and a novel and a familiar object during T2. Control mice spent an equal time exploring the two identical objects in T1 but more time exploring the novel object in T2. They achieved a discrimination index slightly above 0.3. Scopolamine (20 µg ICV) induced a drastic impairment of learning. The mice of this group did not discriminate at all between the novel object and the familiar object in the T2 (Fig. 6). The exploratory activity was not impaired since the exploration time showed no significant difference to that of vehicle-treated control mice (not shown). SCT (2 µg ICV) significantly increased the discrimination index indicating amelioration of the scopolamine-induced impairment of object recognition (Fig. 6).

Figure 6: Effect of scopoletin (SCT, 2 µg ICV) in the object recognition task on the scopolamine-induced (20 µg ICV) learning impairment. The discrimination index is inferior under influence of scopolamine but is significantly ameliorated by SCT. Data are means ± s.e.m., students t-test (* p<0.05).

### 2.2.2 Effect of SCT on T-maze alternation

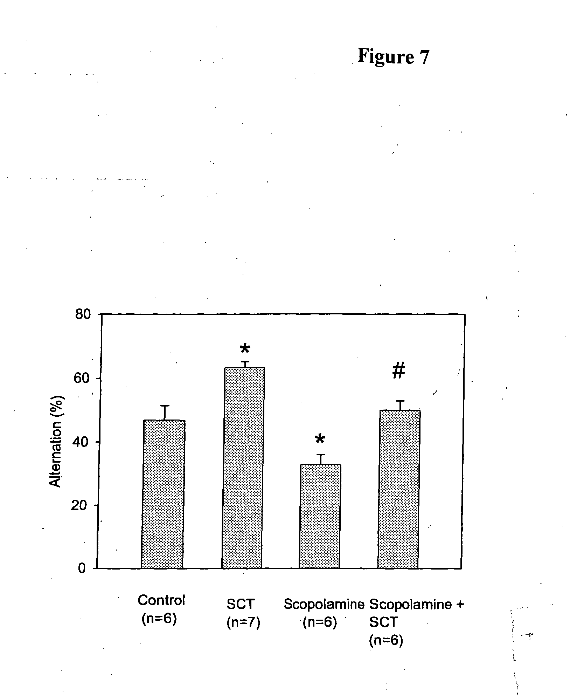

Each mouse was subjected to one forced choice trial followed by the free choice trials. Control mice selected the unfamiliar arm of the maze at a rate not significantly different from chance. However, mice injected with 2 µg SCT showed an increased alternation rate of 63 ± 5 % which indicates a preference for entering the unfamiliar maze arm. These results suggest that SCT facilitates the formation of the spatial working memory which is required to recognize the familiar part of the maze. In contrast to SCT, the anticholinergic drug scopolamin induced a pronounced decrease in alternation rate. SCT abolished this effect of Scopolamine (Fig.7).

Figure 7: Effect of scopoletin (SCT) on spontaneous alternation in the T-maze. SCT (2 µg ICV) increased the alternation rate and abolished the scopolamine-induced (20 µg ICV) memory impairment. Means ± s.e.m., analysis of variance followed by LSD test. * different from control, # different from scopolamine, p<0.05.

### 2.2.3 Effect of SCT on open field locomotor behaviour

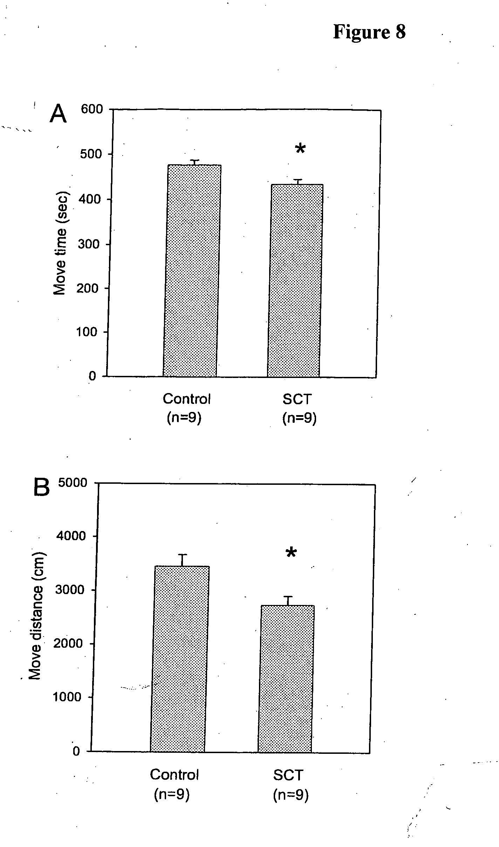

Though SCT showed no significant influence on the locomotion indexes in the learning and memory tests, the coumarin exerted a weak inhibitory effect on locomotor activity in the open field. While the move time of SCT-treated mice was only slightly decreased, the move distance showed about 20 % reduction when compared to that of vehicle-treated controls (Fig. 8 A, B).

Figure 8: Effect of scopoletin (SCT) on A) move time and B) move distance in the open field paradigm. SCT (2 µg ICV) decreased move distance substantially while move time was only marginally reduced. Means ± s.e.m., students t-test (* p<0.05).

The main findings of the study are that the coumarin SCT ameliorates performance of mice in learning tasks in a way that is consistent with its presumed effect to inhibit brain AChE.

In our study, we used the object discrimination task. This paradigm reveals the ability of the animals to recognize the objects they have seen only once during the training trial. Normal mice have a natural propensity to explore novel objects, and when this bias is observed it is inferred that mice remember the familiary object. This kind of object recognition is based on episodic memory, a form of memory that is primarily affected in senile dementia and age-associated impairment and the Alzheimer type of dementia. Also in rats and mice, object discrimination appears to depend on the integrity of the cholinergic system. Our results indicate that object memory in mice is nearly abolished by ICV injection of 20µg scopolamine. We show also that even this strong inhibition by the anticholinergic is partially reversed by 2µg SCT, suggesting a potent action on the brain cholinergic system.

SCT was also effective in the alternation task. When placed in a T-maze, rats or mice possess a strong tendency of alternating arm choices on successive trials. The exploration of novel environmental stimuli is dependent on the integrity of limbic and non-limbic pathways, including the basal forebrain, the hippocampus, the thalamus, the prefrontal cortex, and the dorsal striatum. The spontaneous alternation test is sensitive to the consequences of normal and pathological aging. Neurochemical pathways using acetylcholine, gamma-amino-butyric acid, and dopamine in the septum and hippocampus have been implicated in the exploration of novel maze arms. Spontaneous alternation rates have often been shown to be decreased by drugs which impair cholinergic transmission In contrast, AChE inhibitors increase alternation rate in untreated and pirenzepine-impaired animals. Consistent with these reported effects of AChE inhibitors, SCT enhanced the alternation rate in untreated mice and reversed the scopolamine-induced decrease to control levels.

In the open field paradigm SCT exerted a marginal decrease in move time while the move distance was reduced by about 20%. However, the locomotor indexes of the object memory test and of the alternation test were not significantly influenced by SCT, suggesting that the outcome of performance in these tests depended on learning ability and not on unspecific influences. It has been shown that manipulations of the cholinergic brain systems affect open field behaviour. At higher doses, anticholinergic drugs and inhibition of brain AChE increase locomotor activity and cause hyperlocomotion in the open field. In contrast, AChE inhibitors reduce spontaneous locomotor activity and counteract the stimulatory effect of antagonists such as scopolamine. The mechanism implicated in scopolamine-induced hyperactivity, which is probably related to a dopaminergic system interaction, is different from the mechanism implicated in the scopolamine-induced memory deficit.

### REFERENCES

1. Bores, G. M.; Huger, F. P.; Petko, W.; Mutlib, A. E.; Camacho, F.; Rush, D. K.; Selk, D. E.; Wolf, V.; Kosley, R. W. Jr.; Davis, L; Vargas, H. M. Pharmacological evaluation of novel Alzheimer's disease therapeutics: acetylcholinesterase inhibitors related to galanthamine. *J. Pharmacol. Exp. Ther.* **1996,** 277, 728-738.
2. Damsma, C.; Westerink, B. H. C.; Imperato, A.; Rollema, H.; De Vries, J. B.; Horn, A. S. Automated brain dialysis of acetylcholine in freely moving rats: detection of basal acetylcholine. *Life Sci*. **1987,** 873-876.
3. Ellman, G. L.; Courtney, D.; Andres Jr, V.; Featherstone, R. M. A new rapid calorimetric determination of acetylcholinesterase activity *Biochem. Pharmacol.* **1961,** 7, 88-95.
4. Ganzera, M.; Sturm, S.; Stuppner, H. HPLC-MS and MECC analysis of coumarins. *Chromatographia* **1997,** *46,* 197-203.
5. Ingkaninan, K.; de Best, C. M.; van der Heijeden, R. ; Hofte, A. J. P.; Karabatak, B.; Irth, H.; van der Greef, J.; Verpoorte, R. High-performance liquid chromatography with on-line coupled UV, mass spectrometric and biochemical detection for identification of acetylcholinesterase inhibitors from natural products. *J. Chromatogr. A* **2000,** *872,* 61-73.
6. Lopez, S.; Bastida, J.; Viladomat, F.; Codina, C. Acetylcholinesterase inhibitory activity of some Amaryllidaceae alkaloids and Narcissus extracts. *Life Sciences* **2002,** 71, 2521-2529.
7. Nowak, S.; Dzido, T. H.; Soczewinski, E.; Wolbis, M. Quantitative determination of coumarins, flavonoids and chlorogenic acid in the leaves and underground parts of some species of genus *Scopolia* Jacq. *Acta Pol. Pharm.* **2002,** 59(4), 259-263.
8. Paxinos, G.; Watson, H.; *The rat brain in stereotaxic coordinates;* 4^{th} edn, Academic press: Sidney, **1998.**
9. Prast, H.; Tran, M. H.; Fischer, H.; Philippu, A. Nitric Oxide-induced release of acetylcholine in the nucleus accumbens: role of cyclic GMP, glutamate, and GABA. *J. Neurochem*. **1998,** 77, 266-273.
10. Rhee, I. K.; van de Meent, M.; Ingkaninan, K.; Verpoorte, R. Screening for acetylcholinesterase inhibitors from Amaryllidaceae using silica gel thin-layer chromatography in combination with bioactivity staining. *J. Chromatogr. A* **2001,** *915,* 217-223.
11. Rhee, K.; van Rijn, R. M., Verpoorte, R. Qualitative determination of false-positive effects in the acetylcholinesterase assay using thin layer chromatography. *Phytochem. Anal.* **2003,** *14,* 127-131.
12. Wagner, H.; Bladt, S.; Zgainski, E. M. *Drogenanalyse, Dünnschicht-chromatographische Analyse von Arzneidrogen;* Springer Verlag: Berlin, **1983;** pp. 145-161.
13. Franklin, K. B. J. and Paxinos, G.: The mouse brain in stereotaxic coordinates. San Diego: *Academic Press;* **1997.**
14. Ennaceur A., and Delacour J.; A new one-trial test for neurobiological studies of memory in rats. 1: Behavioural data. *Behav Brain Res.* **1988**, 31:47-59.
15. R. Gerlai; A new continuous alternation task in T-maze detects hippocampal dysfunction in mice. A strain comparison and lesion study. *Behav. Brain Res.* **1998**, 95: 91-101
16. Prut L., Belzung C.; The open field as a paradigm to measure the effects of drugs on anxiety-like behaviors: a review. *Eur J Pharmacol.* **2003,** 463:3-33.

## Claims

1. Use of a coumarin derivative having a coumarin skeleton of formula I 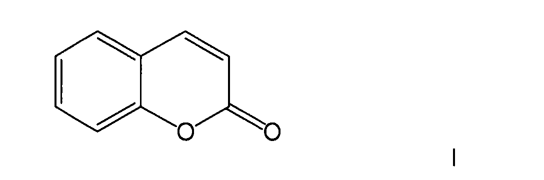 wherein the coumarin skeleton is substituted by one OR¹ and one OR²
whereby
R¹ is C₁ to C₆ alkyl, C₃ to C₇ cycloalkyl or C₂ to C₆ alkenyl and
R² is hydrogen or a sugar moiety,
or a pharmaceutically acceptable salt thereof,
for the preparation of a medicament for the prophylaxis or treatment of disorders which involve low levels of acetylcholine in the brain.

2. Use according to claim 1 for the treatment of neurological and neuromuscular disorders.

3. Use according to claim 1 or 2 for the treatment of a disorder selected from the group consisting of Alzheimer's disease, senile dementia, ataxia, myasthenia gravis and Parkinson's disease.

4. Use according to any of claims 1 to 3 wherein R¹ is selected from C₁ to C₄ alkyl.

5. Use according to any of claims 1 to 4, wherein R² is selected from hydrogen or β-D-glucopyranose.

6. Use according to any of claims 1 to 5, wherein the coumarin derivative has the following formula I(b) 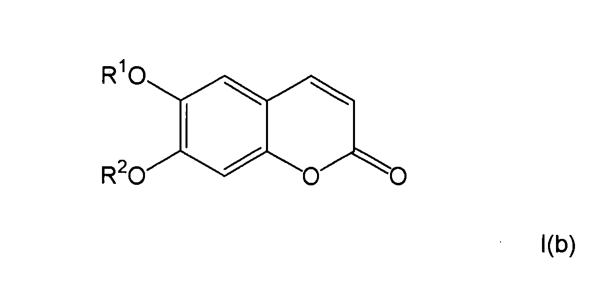 wherein OR¹ and OR² are as defined in claims 1, 4 or 5.
